(19) **Europäisches Patentamt / European Patent Office / Office européen des brevets**

(11) **EP 1 450 880 B1**

(12) **EUROPÄISCHE PATENTSCHRIFT**

(45) Veröffentlichungstag und Bekanntmachung des Hinweises auf die Patenterteilung:
**18.07.2007 Patentblatt 2007/29**

(21) Anmeldenummer: **02792789.6**

(22) Anmeldetag: **22.11.2002**

(51) Int Cl.:
*A61M 1/34* (2006.01) *A61M 1/36* (2006.01)

(86) Internationale Anmeldenummer:
**PCT/EP2002/013115**

(87) Internationale Veröffentlichungsnummer:
**WO 2003/047656 (12.06.2003 Gazette 2003/24)**

(54) **EINRICHTUNG ZUR ÜBERWACHUNG DER ZUFUHR VON SUBSTITUTIONSFLÜSSIGKEIT WÄHREND EINER EXTRAKORPORALEN BLUTBEHANDLUNG**

DEVICE FOR MONITORING THE SUPPLY OF SUBSTITUTION LIQUID DURING AN EXTRACORPOREAL BLOOD TREATMENT

DISPOSITIF POUR CONTROLER L'ACHEMINEMENT DE LIQUIDE DE SUBSTITUTION AU COURS D'UN TRAITEMENT SANGUIN EXTRACORPOREL

(84) Benannte Vertragsstaaten:
**AT BE BG CH CY CZ DE DK EE ES FI FR GB GR IE IT LI LU MC NL PT SE SK TR**

(30) Priorität: **05.12.2001 DE 10159620**

(43) Veröffentlichungstag der Anmeldung:
**01.09.2004 Patentblatt 2004/36**

(73) Patentinhaber: **Fresenius Medical Care Deutschland GmbH**
**61352 Bad Homburg v.d.H. (DE)**

(72) Erfinder:
• **ZHANG, Wei**
**97421 Schweinfurt (DE)**

• **WAMSIEDLER, Ralf**
**97469 Gochsheim-Weyer (DE)**

(74) Vertreter: **Luderschmidt, Schüler & Partner Patentanwälte,**
**Postfach 3929**
**65029 Wiesbaden (DE)**

(56) Entgegenhaltungen:
**EP-A- 0 189 561 EP-A- 0 834 329**
**EP-A- 1 095 666 US-A- 5 366 630**
**US-A- 5 906 589 US-A- 6 090 048**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**EP 1 450 880 B1**

## Beschreibung

Die Erfindung betrifft eine Vorrichtung zur extrakorporalen Blutbehandlung mit einer Einrichtung zur Überwachung der Zufuhr von Substitutionsflüssigkeit während einer extrakorporalen Blutbehandlung.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt das Blut des Patienten durch die Blutkammer. Um das Blut effektiv von den harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinmolekularen Substanzen durch die Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators (Prädilution) oder stromab des Dialysators (Postdilution) dem extrakorporalen Blutkreislauf zugeführt wird.

Es sind Vorrichtungen zur Hämo(dia)filtration bekannt, bei denen die Dialysierflüssigkeit online aus Frischwasser und Konzentraten und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf von dem Flüssigkeitssystem der Maschine über eine Substitutionsflüssigkeitsleitung zugeführt. Bei der Prädilution führt die Substitutionsflüssigkeitsleitung zu einer Anschlußstelle an der arteriellen Blutleitung stromauf des Dialysators, während bei der Postdilution die Substitutionsflüssigkeitsleitung zu einer Anschlußstelle an der venösen Blutleitung stromab des Dialysators führt. Die Substitutionsflüssigkeitsleitung weist einen Konnektor auf, mit dem sie entweder an die venöse oder arterielle Blutleitung angeschlossen werden kann. Zum Unterbrechen der Flüssigkeitszufuhr ist an der Substitutionsflüssigkeitsleitung eine Klemme oder dgl. vorgesehen.

Der korrekte Anschluß der Substitutionsflüssigkeitsleitung wird bei den bekannten Hämo(dia)filtrationsvorrichtungen zu Beginn der Blutbehandlung routinemäßig überprüft. Hierzu werden die zu der Dialysierflüssigkeitskammer führende und von der Dialysierflüssigkeitskammer des Dialysators abgehende Leitung sowie die venöse Blutleitung stromab der Anschlußstelle für die Substitutionsflüssigkeitsleitung mittels Schlauchklemmen abgeklemmt. Die arterielle Blutleitung ist bereits durch die stillstehende Blutpumpe stromauf der Anschlußstelle für die Substitutionsflüssigkeitsleitung unterbrochen. Daraufhin wird die Substituatpumpe zur Förderung der Substitutionsflüssigkeit in Betrieb gesetzt, und der Druck in der venösen Blutleitung wird mittels eines venösen Drucksensors gemessen.

Für den Fall, dass sich mit der Substituatpumpe kein Druck in der venösen Blutleitung aufbauen lässt, der größer als ein vorgegebener Grenzwert ist, wird daraufgeschlossen, dass der Anschluss der Substitutionsflüssigkeitsleitung nicht korrekt, d. h. die Zufuhr von Flüssigkeit unterbrochen ist.

Während der Blutbehandlung kann es in der Dialysepraxis vorkommen, dass das Behandlungsverfahren zwischen Post- und Prädilution gewechselt wird. Hierzu wird die Klemme an der Substitutionsflüssigkeitsleitung geschlossen, und die Substitutionsflüssigkeitsleitung wird von der venösen bzw. arteriellen Blutleitung abgetrennt und an die arterielle bzw. venöse Blutleitung angeschlossen. Dabei ist in der Praxis nicht auszuschließen, dass das Öffnen der Schlauchklemme vergessen wird. Falls die Substituatpumpe nicht gestoppt und der zuvor beschriebene Drucktest nicht durchgeführt werden, wird dieser Zustand nicht erkannt. Nachteilig ist, dass bei den bekannten Hämo(dia)filtrationsvorrichtungen die Zufuhr von Substitutionsflüssigkeit während der Behandlung nicht überwacht wird. Daher bleibt eine Unterbrechung der Substituatzufuhr unerkannt. In diesem Fall kann während einer HDF-Behandlung nur eine wenig effektive Hämodialyse durchgeführt werden. Während einer HF-Behandlung wird der Patient dann im Extremfall gar nicht behandelt. Dies kann für den Patienten mehr oder weniger schwere Folgen haben, obwohl er nicht unmittelbar gefährdet ist.

Aus der EP-A-1 095 666 ist eine Vorrichtung zur extrakorporalen Blutbehandlung bekannt, die eine Zufuhr von Substitutionsflüssigkeit stromauf und stromab des Dialysators vorsieht. Bei der bekannten Blutbehandlungsvorrichtung wird der Druck mittels mehrerer Sensoren in der Blut- und Filtratleitung überwacht, um die Substituatmenge auf der Grundlage der ermittelten Druckwerte zu regeln.

Der Erfindung liegt die Aufgabe zugrunde, eine Vorrichtung bereit zu stellen, die eine Überwachung der Substitutionsflüssigkeitszufuhr auch beim Wechsel des Behandlungsverfahrens ermöglicht.

Die Lösung dieser Aufgabe erfolgt erfindungsgemäß mit den Merkmalen des Patentanspruchs 1.

Die Überwachung der Zufuhr von Substitutionsflüssigkeit beruht darauf, dass die Amplitude der Druckwellen der Substituatpumpe im Flüssigkeitssystem oder extrakorporalen Blutkreislauf überwacht wird. Es hat sich gezeigt, dass eine Störung der Substitutionsflüssigkeitszufuhr dann vorliegt, wenn die Amplitude der Druckwellen einen vorgegebenen Grenzwert überschreitet.

Die erfindungsgemäße Vorrichtung setzt voraus, dass die Substituatpumpe zum Fördern der Substitutionsflüssigkeit eine Druckwellen erzeugende Pumpe, insbesondere eine volumetrische Okklusionspumpe (Rollenpumpe) ist.

Die von der Substituatpumpe ausgehenden Druckwellen können sich über das Flüssigkeitssystem der extrakorporalen Blutbehandlungsvorrichtung durch den Dialysator bis in den extrakorporalen Blutkreislauf fortpflanzen. Dieser Weg ist selbst dann offen, wenn die Substitutionsflüssigkeitszufuhr unterbrochen ist. Dann arbeitet die Substituatpumpe gegen den Verschluss an. Dadurch steigt die Amplitude der Druckwellen, die im extrakorporalen Blutkreislauf detektiert werden können.

Bei einer ersten Ausführungsform wird die Amplitude der Druckwellen im extrakorporalen Blutkreislauf stromab des Dialysators oder Filters gemessen. Dies hat den Vorteil, dass ein Drucksensor verwendet werden kann, der bei den bekannten Blutbehandlungsvorrichtungen ohnehin in der venösen Blutleitung vorgesehen ist.

Bei einer alternativen Ausführungsform wird die Amplitude der Druckwellen im Flüssigkeitssystem stromauf des Dialysators oder Filters gemessen. Dies hat den Vorteil, dass eine Überwachung auch beim Betrieb der Blutbehandlungsvorrichtung als Hamofiltrationsvorrichtung möglich ist, bei dem die Druckwellen den extrakorporalen Kreislauf nicht erreichen können, da der Einlass des Dialysator bzw. Filters von der Dialysierflüssigkeitszufuhr abgetrennt ist.

Das Drucksignal wird vorzugsweise zur Eliminierung von Störimpulsen mit einem Bandpaß gefiltert, wobei die Amplitude des gefilterten Drucksignals dann mit dem vorgegebenen Grenzwert verglichen wird.

Es hat sich gezeigt, daß die Vergrößerung der Amplitude der Druckwellen abhängig von der Pumprate (Drehzahl) der Substituatpumpe ist. Ein Fehlalarm kann dadurch ausgeschlossen werden, daß bei Überschreiten des vorgegebenen Grenzwertes nur dann auf einen Störfall geschlossen wird, wenn die Pumprate zwischen vorgegebenen Grenzwerten liegt. Diese Grenzwerte sollten so bemessen sein, daß die auf die Änderungen der Pumprate zurückzuführende Amplitudenänderung geringer als die Amplitudenänderung infolge einer Unterbrechung der Substitutionsflüssigkeitszufuhr ist.

Zweckmäßigerweise wird bei Überschreiten des vorgegebenen Grenzwertes ein akustischer und/oder optischer Alarm gegeben. Es kann aber auch ein Eingriff in die Steuerung der Blutbehandlungsvorrichtung vorgenommen werden.

Die Überwachungseinrichtung der extrakorporalen Blutbehandlungsvorrichtung weist Mittel zur Überwachung der Amplitude der Druckwellen der Substituatpumpe im Flüssigkeitssystem, vorzugsweise stromauf des Dialystors oder Filters oder im extrakorporalen Blutkreislauf, vorzugsweise stromab des Dialysators oder Filters, und Mittel zum Auswerten der Druckwellenamplitude auf. Die Druckwellen erzeugende Substituatpumpe ist in der Substitutionsflüssigkeitsleitung angeordnet, die von dem Flüssigkeitssystem zu dem Blutkreislauf stromauf oder stromab des Dialysators oder Filters führt. Die Substitutionsflüssigkeitsleitung kann eine einzige Schlauchleitung oder auch ein Schlauchleitungssystem mit mehreren Zweigen sein.

Allein entscheidend ist, daß eine Strömungsverbindung zwischen dem Flüssigkeitssystem und dem Blutkreislauf der Blutbehandlungsvorrichtung hergestellt wird.

Die Substitutionsflüssigkeitsleitung kann direkt an der venösen oder arteriellen Blutleitung angeschlossen werden. Es ist aber auch möglich, daß die Leitung an Tropfkammern oder dgl. angeschlossen wird, die in der venösen bzw. arteriellen Blutleitung vorgesehen sind.

Das Flüssigkeitssystem der Blutbehandlungsvorrichtung kann eine zu der Dialysierflüssigkeitskammer des Dialysators führende Dialysierflüssigkeitszufuhr- und eine von der Dialysierflüssigkeitskammer des Dialysators abgehende Dialysierflüssigkeitsabfuhrleitung umfassen. In dem Flüssigkeitssystem können ein oder mehrere Filter zur Erhöhung der Sicherheit angeordnet sein. Auch können weitere Leitungen, beispielsweise Bypass-Leitungen etc. vorgesehen sein. Allein entscheidend ist, daß das Flüssigkeitssystem eine Fortpflanzung von Druckwellen erlaubt, d. h. ein vollständig mit einem Medium gefülltes System ist.

Im folgenden wird ein Ausführungsbeispiel der Erfindung unter Bezugnahme auf die Zeichnungen näher erläutert. Es zeigen:

Figur 1   eine Vorrichtung zur extrakorporalen Blutbehandlung mit einer Einrichtung zur Überwachung der Zufuhr von Substitutionsflüssigkeit in stark vereinfachter schematischer Darstellung,

Figur 2 (A) - (C)   das gefilterte venöse Drucksignal als Funktion der Zeit bei Postdilution (A), Diskonnektion (B) und Prädilution (C), wenn die Pumpenrate der Substituatpumpe 20 ml/min beträgt,

Figuren 3 (A) - (C)   das gefilterte venöse Drucksignal bei einer Substituatpumpenrate von 60 ml/min., und

Figuren 4 (A) - (C)   das gefilterte venöse Drucksignal bei einer Substituatpumpenrate von 100 ml/min.

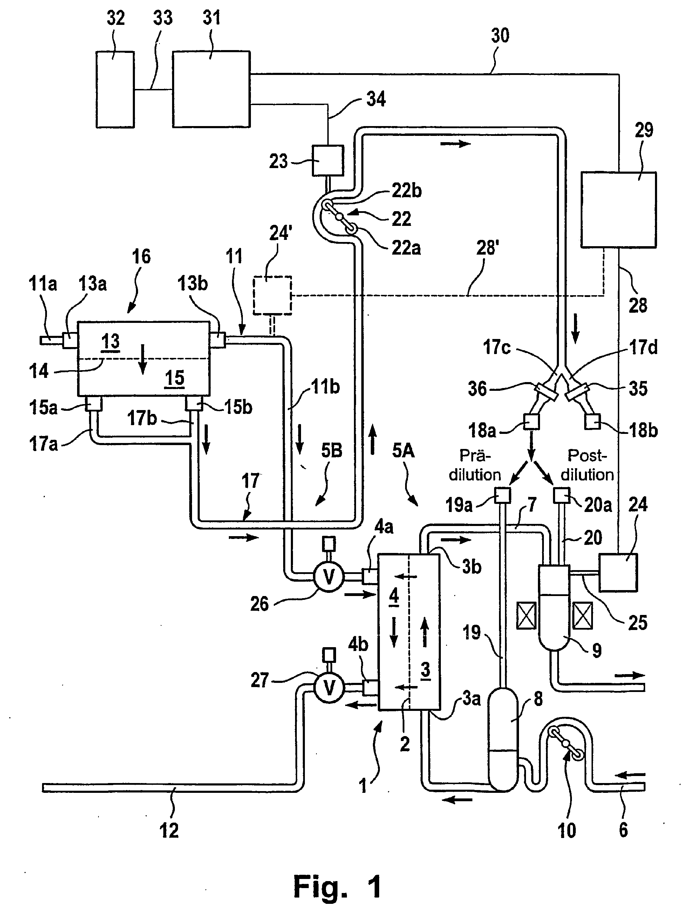

Figur 1 zeigt eine vereinfachte schematische Darstellung der wesentlichen Komponenten einer Hämo(dia)filtrationsvorrichtung zusammen mit einer Einrichtung zur Überwachung der Zufuhr von Substitutionsflüssigkeit aus dem Flüssigkeitssystem der Hamö(dia)filtrationsvorrichtung in den extrakorporalen Blutkreislauf.

Die Hämo(dia)filtrationsvorrichtung weist einen Dialysator oder Filter 1 auf, der durch eine Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist.

Der extrakorporale Blutkreislauf 5A umfaßt eine arterielle Blutleitung 6, die zu dem Einlaß 3a der Blutkammer 3 führt, und eine venöse Blutleitung 7, die von dem Auslaß 3b der Blutkammer 3 des Dialysators 1 abgeht. Zur Eliminierung von Luftblasen sind in die arterielle Blutleitung 6 eine arterielle Tropfkammer 8 und in die venöse Blutleitung 7 eine venöse Tropfkammer 9 geschaltet. Das Blut des Patienten wird durch die Blutkammer des Dialysators mittels einer arteriellen Blutpumpe 10, insbesondere Rollenpumpe gefördert, die an der arteriellen Blutleitung 6 angeordnet ist.

Das Flüssigkeitssystem 5B umfaßt eine Dialysierflüssigkeitszuführleitung 11, die zu dem Einlaß 4a der Dialysierflüssigkeitskammer 4 führt, und eine Dialysierflüssigkeitsabführleitung 12, die von dem Auslaß 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer, während die verbrauchte Dialysierflüssigkeit aus der Dialysierflüssigkeitskammer über die Dialysierflüssigkeitsabführleitung 12 zu einem nicht dargestellten Abfluß abgeführt wird. Die in den Hämo(dia)filtrationsvorrichtungen im allgemeinen vorgesehene Bilanziereinrichtung zur Bilanzierung frischer gegen verbrauchte Dialysierflüssigkeit ist der besseren Übersichtlichkeit halber nicht dargestellt. Auch sind zusätzliche Einrichtungen zum Reinigen und Spülen des Systems nicht dargestellt.

Die Dialysierflüssigkeitszuführleitung 11 umfaßt einen ersten Abschnitt 11a, der zu dem Einlaß 13a einer ersten Kammer 13 eines durch eine Membran 14 in die erste Kammer und eine zweite Kammer 15 unterteilten Sterilfilters 16 führt und einen zweiten Abschnitt 11b, der von dem Auslaß 13b der ersten Kammer 13 des Filters 16 abgeht und zu dem Einlaß 4a der Dialysierflüssigkeitskammer 4 führt.

Während der Dialysebehandlung kann Dialysierflüssigkeit aus dem Flüssigkeitssystem 5B als Substitutionsflüssigkeit über die Schlauchleitung 17 dem extrakorporalen Blutkreislauf 5A zugeführt werden. Die Substitutionsflüssigkeitsleitung 17 weist an beiden Enden jeweils zwei Leitungsabschnitte 17a, 17b, 17c, 17d auf. Der Leitungsabschnitt 17a ist mit dem einem ersten Auslaß 15a und der Leitungsabschnitt 17b mit einem zweiten Auslaß 15b der zweiten Kammer 15 des Sterilfilters 16 verbunden, während an den Leitungsabschnitten 17c und 17d jeweils ein Konnektor 18a, 18b angeschlossen sind. Mit den beiden Konnektoren 18a, 18b wird die Substitutionsflüssigkeitsleitung 17 an eine zu der arteriellen Tropfkammer 8 führende Anschlußleitung 19 und eine zu der venösen Tropfkammer 9 führende Anschlußleitung 20 angeschlossen. Die Anschlußleitungen 19, 20 verfügen dafür über entsprechende Anschlußstücke 19a, 20a. An den Leitungsabschnitten 17c und 17d sind Schlauchklemmen 35, 36 vorgesehen, mit denen wahlweise eine Flüssigkeitsverbindung mit der Anschlußleitung 19 oder 20 hergestellt werden kann, um eine Prä- oder Postdilution vorzunehmen. Auf eine Abzweigung kann aber auch verzichtet werden, wenn zum Abklemmen der Substitutionsflüssigkeitsleitung 17 stromab der Substituatpumpe 22 eine Schlauchklemme vorgesehen ist. Dann ist es aber erforderlich, die Leitungsanschlüsse manuell zu tauschen.

Die Substitutionsflüssigkeit wird mittels einer Okklusionspumpe, insbesondere Rollenpumpe 22 gefördert, in die die Substitutionsflüssigkeitsleitung 17 eingelegt ist. Derartige Rollenpumpen gehören zum Stand der Technik. Sie verfügen über mehrere Rollen 22a, 22b, mit denen der Querschnitt der Schlauchleitung zur Förderung der Flüssigkeit verringert wird. Dadurch entstehen Druckwellen, die sich in beiden Richtungen über die Substitutionsflüssigkeitsleitung fortpflanzen können. An der Substituatpumpe 22 ist ein Hall-Sensor 23 vorgesehen, der die Pumpenrate mißt.

Zur Messung des Drucks in der venösen Blutleitung 7 ist ein Drucksensor 24 vorgesehen, der über eine Druckleitung 25 an der venösen Tropfkammer 9 angeschlossen ist. Der Drucksensor liefert ein dem Druck in der venösen Blutleitung proportionales elektrisches Signal.

Zum Betrieb der Hämo(dia)filtrationsvorrichtung als Hämodialysevorrichtung werden die Schlauchklemmen 35, 36 geschlossen, so daß Dialysierflüssigkeit durch die Dialysierflüssigkeitskammer 4 des Dialysators strömt. Zum Betrieb der Hämo(dia)filtrationsvorrichtung als Hämodiafiltrationsvorrichtung wird die Schlauchklemme 35, 36 geöffnet, so daß aus dem Sterilfilter 16 sterile Dialysierflüssigkeit als Substitutionsflüssigkeit in die venöse Tropfkammer 8 (Prädilution) oder arterielle Tropfkammer 9 (Postdilution) strömt. Es ist aber auch ein Betrieb der Hämo(dia)filtrationsvorrichtung nur als Hämofiltrationsvorrichtung möglich, wenn der Zufluß von Dialysierflüssigkeit in die Dialysierflüssigkeitskammer 4 des Dialysators 1 unterbrochen wird. Zur Unterbrechung der Flüssigkeitszufuhr ist stromauf des Dialysators 1 ein Absperrorgan 26 vorgesehen.

Der venöse Drucksensor 24 ist mit einer Signalleitung 28 mit einem Bandpaßfilter 29 verbunden. Der Bandpaßfilter 29 wiederum ist mit einer Datenleitung 30 mit einer Auswerteinheit 31 verbunden, die über eine weitere Signalleitung 34 von dem Hall-Sensor 23 ein von der Pumprate der Substituatpumpe 22 abhängiges elektrisches Signal empfängt.

Die Auswerteinheit 31 bestimmt die Amplitude des mit dem Bandpaßfilter 29 gefilterten Drucksignals und vergleicht die Amplitude mit einem vorgegebenen Grenzwert. Als vorgegebener Grenzwert wird das 1,5- bis 2,5-fache, vorzugsweise das 1,8- bis 2,2-fache, insbesondere das 2,0-fache der bei störungsfreiem Betrieb gemessenen Amplitude der Druckwellen angenommen.

Für den Fall, daß nach einem Wechsel der Behandlung beispielsweise von Präauf Postdilution das Öffnen der Schlauchklemme 35, 36 vergessen wird, d. h. die Substitutionsflüssigkeitsleitung 17 abgeklemmt ist, steigt die Amplitude des Drucksignals stark an.

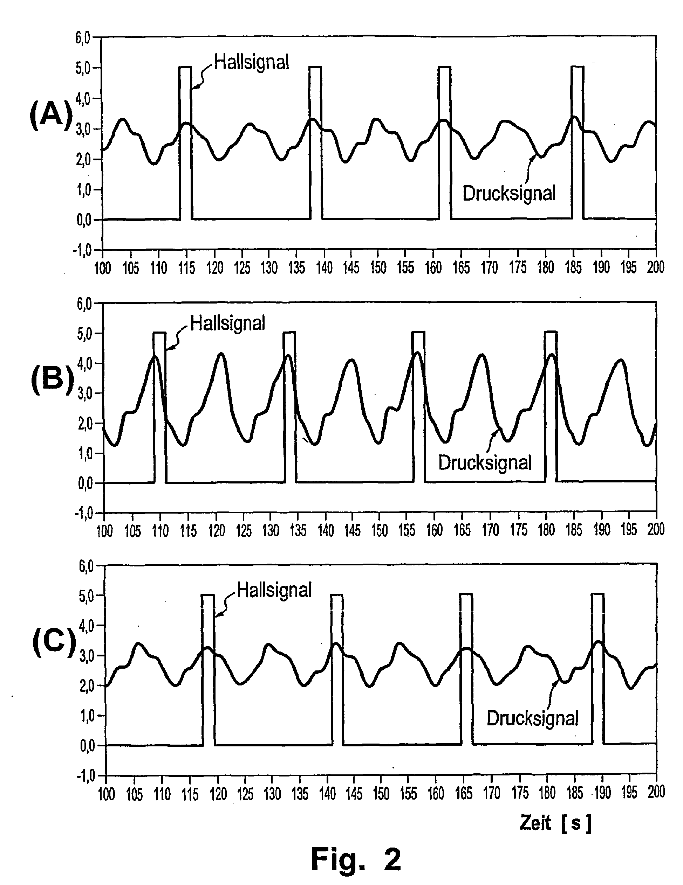

Die Figuren 2A, 2B und 2C zeigen das gefilterte venöse Drucksignal zusammen mit dem periodischen Signal des Hall-Sensors als Funktion der Zeit bei einer Substituatpumpenrate von 20 ml/min für die Fälle Postdilution (A), Diskonnektion (B) und Prädilution (C). Blut- und Dialysatfluß sind auf 300 ml/min eingestellt. Es ergeben sich folgende Werte:

$$Ampl_{post} = 1,5 \text{ V}; \; Ampl_{discon} = 3,0 \text{ V}; \; Ampl_{pre} = 1,5 \text{ V}.$$

$$Ampl_{discon} / Ampl_{post} = 2,0; \; Ampl_{discon} / Ampl_{pre} = 2,0$$

Es zeigt sich, daß bei einer Unterbrechung des Substitutionsflüssigkeitsflusses die Amplitude der Druckwelle verdoppelt ist. Wenn also die Auswerteinheit feststellt, daß die Druckamplitude größer als der vorgegebene Grenzwert ist, beispielsweise das 2,0-fache des Normalwertes, erzeugt die Auswerteinheit ein Alarmsignal, das eine Alarmeinheit 32 über eine Alarmleitung 33 empfängt. Die Alarmeinheit 32 gibt dann einen akustischen und/oder optischen Alarm.

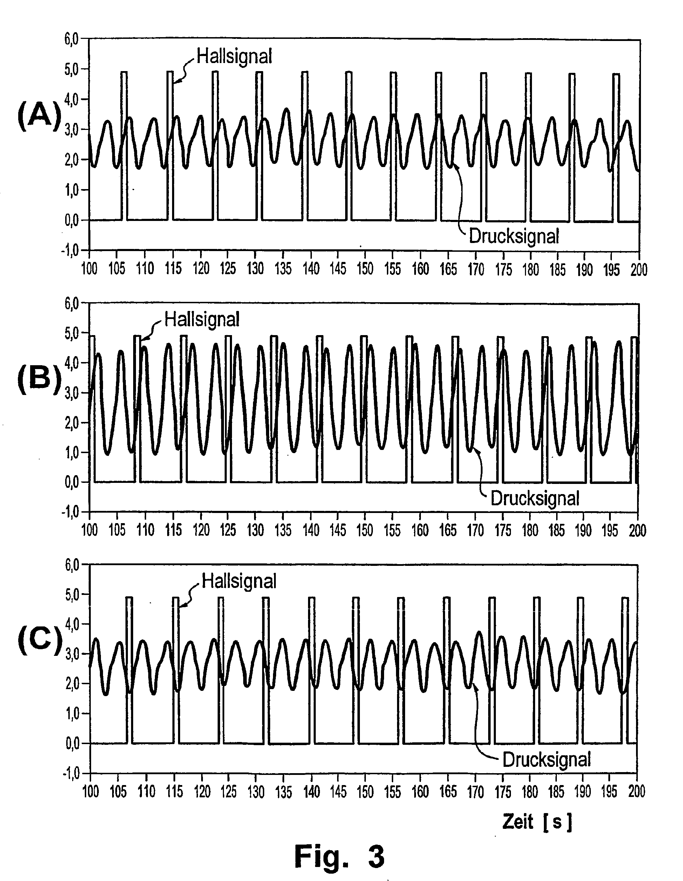

Die Figuren 3A, 3B und 3C zeigen das gefilterte venöse Drucksignal und das Hall-Signal als Funktion der Zeit bei einer höheren Pumprate von 60 ml/min bei Postdilution (A), Diskonnektion (B) und Prädilution (C). Blut- und Dialysatfluß sind wieder 300 ml/min. Es ergeben sich folgende Werte:

$$Ampl_{post} = 1,9 \text{ V}; \; Ampl_{discon} = 3,6 \text{ V}; \; Ampl_{pre} = 1,8 \text{ V}.$$

$$Ampl_{discon} / Ampl_{post} = 1,9; \; Ampl_{discon} / Ampl_{pre} = 2,0$$

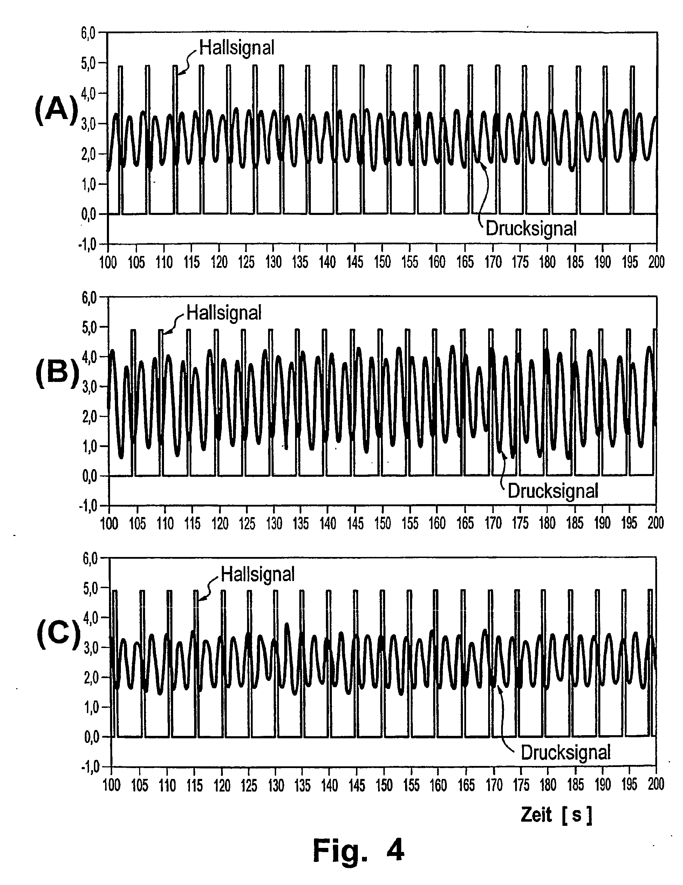

Das gefilterte venöse Drucksignal und das Hall-Signal bei einer noch höheren Pumpenrate von 100 ml/min. bei Postdilution (A), Diskonnektion (B) und Prädilution (C) zeigen die Figuren 4A, 4B und 4C. Blut- und Dialysatfluß sind wieder 300 ml/min. Es ergeben sich folgende Werte:

$$Ampl_{post} = 1,7 \text{ V}; \; Ampl_{discon} = 3,2 \text{ V}; \; Ampl_{pre} = 1,7 \text{ V}.$$

$$Ampl_{discon} / Ampl_{post} = 1,9; \; Ampl_{discon} / Ampl_{pre} = 1,9$$

Zu den Amplitudenwerten der Figuren 2 und 3 sei bemerkt, daß die Meßwerte angepaßt worden sind, um vergleichbare Größen zu haben. In der Praxis ist der Anstieg der Druckamplitude bei Unterbrechung des Zuflusses von Substitutionsflüssigkeit von der Pumprate nicht unabhängig.

Zur Erhöhung der Sicherheit kann ein Fehlalarm dadurch ausgeschlossen werden, daß auch die Pumprate bei dem Vergleich des gemessenen Drucksignals mit dem vorgegebenen Grenzwert berücksichtigt wird. Hierzu empfängt die Auswerteinheit 31 auch das Signal des Hall-Sensores 23.

Solange die Pumprate zwischen vorgegebenen Grenzen liegt, nimmt die Auswerteinheit an, daß ein Anstieg der Druckamplitude eine Folge einer Unterbrechung des Substitutionsflüssigkeitszuflusses ist. Beispielsweise kann als Grenzwert Amplitude$_{nachher}$/Amplitude$_{vorher}$ > 1,5 angenommen werden. Für höhere Pumpraten ist der Schwellwert entspechend zu korrigieren. Dies kann dadurch erfolgen, daß für verschiedene Pumpratenbereiche unterschiedliche Schwellwerte angenommen werden.

Der Anstieg der Druckamplitude ist auf folgendes zurückzuführen. Bei einer Blockierung des Zuflusses von Substitutionsflüssigkeit sind die Federkräfte des Rotors der Rollenpumpe 22 nicht mehr in der Lage, den Substitutionsflüssigkeitsschlauch 17 zusammenzudrücken, so daß der maximale Systemdruck erreicht ist. Dieser Druck, der auch als Okklusionsdruck bezeichnet wird, ist wesentlich höher als der normale Systemdruck. Der Rotor fördert somit bei einer Substitutionsflüssigkeitsblockade gegen den höheren Okklusionsdruck an. Wegen der geschlossenen Schlauchklemme 35 bzw. 36 ist der Übertragungsweg der Druckwellen über den Substitutionsflüssigkeitsschlauch 17 zum venösen Drucksensor 24 zwar vollständig blockiert. Die von der Substituatpumpe 22 erzeugten Druckwellen erreichen den venösen Drucksensor 24 aber über den Sterilfilter 16, den zweiten Abschnitt 11b der Dialysierflüssigkeitszuführleitung, den Dialysator 1, die venöse Blutleitung 7, die venöse Tropfkammer 9 und schließlich die Druckleitung 25. Wie die Figuren 2 bis 4 zeigen, verdoppelt sich die Amplitude des Drucksignals bei Diskonnektion aufgrund des erhöhten Okklusionsdrucks.

Bei einer alternativen Ausführungsform werden die Druckwellen nicht im extrakorporalen Blutkreislauf (5A), sondern im Flüssigkeitssystem (5B) der Blutbehandlungsvorrichtung stromauf des Dialysators 1 oder Filters überwacht. Diese Ausführungsform unterscheidet sich von dem oben beschriebenen Ausführungsbeispiel dadurch, daß nicht der Drucksensor 24 in der venösen Blutleitung 7, sondern ein Drucksensor 24' in dem zweiten Abschnitt 11b der Dialysierflüssigkeitszuführleitung vorgesehen ist. Dieser Drucksensor 24' ist in Fig. 1 in gestrichelten Linien als alternative Ausführungsform angedeutet. Er ist über die ebenfalls in gestrichelten Linien angedeutete Signalleitung 28' mit dem Tiefpaßfilter 29 verbunden. Die Druckwellen können aber auch an einer anderen Stelle des Flüssigkeitssystem gemessen werden.

**Patentansprüche**

1. Vorrichtung zur extrakorporalen Blutbehandlung mit einem extrakorporalen Blutkreislauf (5 A), der eine erste Kammer (3) eines durch eine semipermeable Membran (2) in die erste Kammer und eine zweite Kammer (4) unterteilten Dialysators (1) oder Filters einschließt, und einem Flüssigkeitssystem (5B), das die zweite Kammer des Dialysators oder Filters einschließt, wobei eine Substitutionsflüssigkeitsleitung (17) von dem Flüssigkeitssystem zu dem Blutkreislauf stromauf oder stromab des Dialysators oder Filters führt, in der eine Druckwellen erzeugende Substituatpumpe (22) angeordnet ist, und einer Einrichtung zur Überwachung der Zufuhr von Substitutionsflüssigkeit aus dem Flüssigkeitssystem in den Blutkreislauf, **dadurch gekennzeichnet, dass** die Überwachungseinrichtung Mittel (24), (29) zum Überwachen der Amplitude der Druckwellen der Substituatpumpe (22) im Flüssigkeitssystem (5B) oder extrakorporalen Blutkreislauf (5A) und Mittel (31) zum Auswerten der Druckwellenamplitude aufweist, die derart ausgebildet sind, dass auf eine Störung der Substitutionsflüssigkeitszufuhr dann geschlossen wird, wenn die Amplitude der Druckwellen einen vorgegebenen Grenzwert überschreitet.

2. Vorrichtung nach Anspruch 1, **dadurch gekennzeichnet, dass** die Mittel (24), (29) zum Überwachen der Amplitude der Druckwellen der Substituatpumpe (22) Mittel zum Überwachen der Druckwellen im extrakorporalen Blutkreislauf (5 A) stromab des Dialysators (1) oder Filters sind.

3. Vorrichtung nach Anspruch 1, **dadurch gekennzeichnet, dass** die Mittel (24'), (29) zum Überwachen der Amplitude der Druckwellen der Substituatpumpe (22) Mittel zum Überwachen der Druckwellen im Flüssigkeitssystem (5B) stromauf des Dialysators (1) oder Filters sind.

4. Vorrichtung nach einem der Ansprüche 1 - 3, **dadurch gekennzeichnet, dass** Mittel (23) zum Überwachen der Pumprate der Substituatpumpe (22) vorgesehen sind, wobei die Mittel (31) zum Auswerten der Druckwellenamplitude derart ausgebildet sind, dass auf eine Störung bei Überschreiten des Grenzwertes nur dann geschlossen wird, wenn die Pumprate zwischen vorgegebenen Grenzwerten liegt.

5. Vorrichtung nach einem der Ansprüche 1 - 3, **dadurch gekennzeichnet, dass** die Mittel (24), (29) zum Überwachen der Amplitude der Druckwellen einen Drucksensor (29) zum Messen des Drucks im extrakorporalen Kreislauf (5 A) stromab des Dialysators (1) oder Filters aufweisen, wobei der Drucksensor (24) ein von dem Druck abhängiges Signal erzeugt, und einen Bandpassfilter (29) zum Filtern des Drucksignals aufweisen.

6. Vorrichtung nach einem der Ansprüche 1 - 4, **dadurch gekennzeichnet, dass** eine Alarmeinheit (32) zum Erzeugen eines akustischen und/oder optischen Alarms bei Überschreiten des vorgegebenen Grenzwertes vorgesehen ist.

7. Vorrichtung nach einem der Ansprüche 1 - 5, **dadurch gekennzeichnet, dass** die Substituatpumpe eine Rollenpumpe (22) ist.

**Claims**

1. An apparatus for extracorporeal blood treatment with an extracorporeal blood circuit (5A), which includes a first chamber (3) of a dialyser (1) or filter divided by a semipermeable membrane (2) into the first chamber and a second chamber (4), and a fluid system (5B), which includes the second chamber of the dialyser or filter, a substitution fluid line (17) leading from the fluid system to the blood circuit upstream or downstream of the dialyser or filter, in which a substitute pump (22) generating a pressure wave is disposed, and a device for monitoring the supply of substitution fluid from the fluid system into the blood circuit, **characterised in that** the monitoring device comprises means (24), (29) for monitoring the amplitude of the pressure waves of the substitute pump (22) in the fluid system (5B) or extracorporeal blood circuit (5B) and means (31) for evaluating the pressure wave amplitude, which are designed in such a way that it is concluded that there is a malfunction of the substitution fluid supply when the amplitude of the pressure waves exceeds a preset threshold value.

2. The apparatus according to claim 1, **characterised in that** the means (24), (29) for monitoring the amplitude of the pressure waves of the substitute pump (22) are means for monitoring the pressure waves in the extracorporeal blood circuit (5A) downstream of the dialyser (1) or filter.

3. The apparatus according to claim 1, **characterised in that** the means (24'), (29) for monitoring the amplitude of the pressure waves of the substitute pump (22) are means for monitoring the pressure waves in the fluid system (5B) upstream of the dialyser (1) or filter.

4. The apparatus according to any one of claims 1 - 3, **characterised in that** means (23) are provided for monitoring the pump rate of the substitute pump (22), wherein the means (31) for evaluating the pressure wave amplitude are designed in such a way that it is concluded that there is a malfunction when the threshold value is exceeded only when the pump rate lies between preset threshold values.

5. The apparatus according to any one of claims 1 - 3, **characterised in that** the means (24), (29) for monitoring the amplitude of the pressure waves comprise a pressure sensor (29) for measuring the pressure in the extracorporeal circuit (5A) downstream of the dialyser (1) or filter, wherein the pressure sensor (24) generates a signal dependent on the pressure, and comprise a band-pass filter (29) for filtering the pressure signal.

6. The apparatus according to any one of claims 1 - 4, **characterised in that** an alarm unit (32) is provided for generating an acoustic and/or optical alarm when the preset threshold value is exceeded.

7. The apparatus according to any one of claims 1 - 5, **characterised in that** the substitute pump is a roller pump (22).

**Revendications**

1. Dispositif pour le traitement sanguin extracorporel avec une circulation sanguine extracorporelle (5 A) enfermant une première chambre (3) d'un dialyseur (1) ou d'un filtre partagé par une membrane semiperméable (2) en la première chambre et une deuxième chambre (4), avec un système de liquide (5B) enfermant la deuxième chambre du dialyseur ou du filtre, une conduite de liquide de substitution (17) allant du système de liquide vers la circulation sanguine en amont ou en aval du dialyseur ou du filtre, ladite conduite comportant une pompe de substituat (22) produisant des ondes de pression, et avec un dispositif pour contrôler l'acheminement de liquide de substitution depuis le système de liquide dans la circulation sanguine, **caractérisé en ce que** le dispositif de contrôle comporte des moyens (24, 29) pour contrôler l'amplitude des ondes de pression de la pompe de substituat (22) dans le système de liquide (5B) ou dans la circulation sanguine extracorporelle (5A) et des moyens (31) pour analyser ladite amplitude des ondes de pression, lesdits moyens étant configurés de telle sorte que l'on peut conclure à un dérangement de l'acheminement du liquide de substitution lorsque l'amplitude des ondes de pression excède une valeur limite prédéfinie.

2. Dispositif selon la revendication 1, **caractérisé en ce que** les moyens (24, 29) pour contrôler l'amplitude des ondes de pression de la pompe de substituat (22) sont des moyens pour contrôler les ondes de pression dans la circulation sanguine extracorporelle (5A) en aval du dialyseur (1) ou du filtre.

3. Dispositif selon la revendication 1, **caractérisé en ce que** les moyens (24', 29) pour contrôler l'amplitude des ondes de pression de la pompe de substituat (22) sont des moyens pour contrôler les ondes de pression dans le système de liquide (5B) en amont du dialyseur (1) ou du filtre.

4. Dispositif selon l'une quelconque des revendications 1 - 3, **caractérisé en ce que** des moyens (23) pour contrôler le débit de la pompe de substituat (22) sont prévus, les moyens (31) destinés à l'analyse de l'amplitude des ondes de pression étant configurés de telle sorte que l'on ne peut conclure à un dérangement, lors du dépassement de la valeur limite, que si le débit se situe entre des valeurs limites prédéfinies.

5. Dispositif selon l'une quelconque des revendications 1 - 3, **caractérisé en ce que** les moyens (24), (29) pour contrôler l'amplitude des ondes de pression comportent un capteur de pression (29) prévu pour mesurer la pression dans la circulation extracorporelle (5A) en aval du dialyseur (1) ou du filtre, ledit capteur de pression (24) générant un signal dépendant de la pression, lesdits moyens comportant également un filtre passe bande (29) pour filtrer le signal pression.

6. Dispositif selon l'une quelconque des revendications 1 - 4, **caractérisé en ce qu'**une unité d'alarme (32) est prévue pour générer une alarme acoustique et/ou optique dans le cas d'un dépassement de la valeur limite prédéfinie.

7. Dispositif selon l'une quelconque des revendications 1 - 5, **caractérisé en ce que** la pompe de substituat est une pompe à rouleaux (22).

**Fig. 1**

Fig. 2

Fig. 3

Fig. 4

**EP 1 450 880 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1095666 A